# EUROPEAN PATENT SPECIFICATION

(11) **EP 2 207 528 B1**
(45) Date of publication and mention of the grant of the patent: **09.10.2013**
(21) Application number: 08759177.2
(22) Date of filing: 11.06.2008
(51) Int. Cl.: A61K 9/00

(54) **PHARMACEUTICAL COMPOSITION**
PHARMAZEUTISCHE ZUSAMMENSETZUNG
COMPOSITION PHARMACEUTIQUE

(30) Priority: 05.10.2007 EP 07019558
(43) Date of publication of application: 21.07.2010
(73) Proprietor: STI Pharmaceuticals Ltd., Doddinghurst Brentwood Essex CM15 0QX (GB)
(72) Inventor: DAVIES, Andrew, Brentwood (GB); STEUP, Christian, 65719 Hofheim (DE)
(74) Representative: Pohlmann, Bernd Michael
(86) International application number: PCT/EP2008/004665
(87) International publication number: WO 2009/043395

(56) References cited:
- WO-A-01/89589
- WO-A-2005/079771
- MYRTHA NAEF ET AL: "Development and pharmacokinetic characterization of pulmonal and intravenous delta-9-tetrahydrocannabinol (THC) in humans", JOURNAL OF PHARMACEUTICAL SCIENCES, AMERICAN PHARMACEUTICAL ASSOCIATION, WASHINGTON, US, vol. 93, no. 5, 1 May 2004 (2004-05-01), pages 1176-1184, XP008140183, ISSN: 0022-3549, DOI: 10.1002/JPS.20037 [retrieved on 2004-02-23]

## Description

The current invention relates to a pharmaceutical composition, comprising at least one cannabinoid and an effective amount of an active ingredient for cough suppression as well as the administration of said composition as an aerosol. Further the invention relates to an inhaler, preferably a metered dose inhaler containing said composition Further the invention relates to a method for manufacturing said composition and for administering said composition comprising a cannabinoid and an effective amount of an active ingredient for cough suppression to a patient.

### Background of the invention

There has been a revival in cannabinoid research since the publication of the House of Lords Science & Technology Sub Committee report (The development of Prescription Cannabis-Based Medicines Jan 2001), which called for further research into the therapeutic potential of cannabis derived medications. Cannobinoids include delta-9-Tetrahydrocannabinol (THC), delta-8-Tetrahydrocannabinol, Cannabidiol (CBD), Cannabinol (CBN), tetrahydrocannabinovarin (THCV), cannabidivarin (CBDV), cannabigerol (CBG) and cannabichromene (CBC) and combinations thereof.

The renewed emphasis in cannabinoid research covers a wide range of indications, which continues to expand. The following are some of the indications under clinical development: pain management, bladder dysfunction, anorexia, epilepsy, mood disorders, glaucoma, head trauma, spinal cord injury, breast cancer, inflammatory bowl disorder and spasticity associated with multiple sclerosis.

More recently evidence suggests that CBD may play an important role in the modulation of psychiatric disorders such as anxiety, bipolar, depression and schizophrenia (C.H.Ashton et al (2005);"Cannabinoids in bipolar affective disorder: a review and discussion of their therapeutic potential" Psychopharm 2005).

A common problem in cannabinoid research is the administration of the active ingredients and, thus, a controllable and/or systemic bioavailability.

### Typical routes of delivery

Due to variable absorption and extensive first-pass metabolism, the bioavailability of oral delta-9-tetrahydrocannabinol (THC) is low (5-20%) and therefore alternative delivery forms are necessary.

The following is an overview of some routes of administration and the associated advantages and disadvantages of each.

### Smoking

It is well documented that most of the compounds contained within cannabis and cigarette smoke are harmful. There is evidence to suggest that marijuana smoke produces more tar than cigarettes (Tashkin et al. Tashkin, D.P. Effects of Marijuana on the Lung and its Immune Defences. Indiana Prevention Resource Centre March 1997).

Smoke inhalation, however, as a drug delivery route is highly effective in delivering compounds systemically into the blood.Recorded plasma levels of cannabinoids up to 24%. Smoke particulate inhaled deep into the lung is fine enough to reach the upper alveoli, the site at which blood/gas interchange occurs, resulting in rapid systemic uptake of compounds carried within smoke. Unfortunately such an efficient carrier system also delivers undesirable compounds deep into the lung such as carcinogens.

### Oral medicament

In 1985 the world's first synthetic cannabinoid was licensed in the US for nausea and vomiting associated with chemotherapy and HIV related wasting-disorders. The product, known as Marinol ® contains a synthetic tetrahydocannabidinol (THC) dissolved in sesame oil, sealed in a soft gelatin capsule for oral application.

Although the introduction of Marinol ® was a significant development in cannabinoid research, the oral route of administration leads to variable bioavailability and a time-to-onset typically 0.5 to 2 hours. THC is a lipophilic compound and when administered orally it is subject to the first pass effect i.e. absorbed by the liver and consequently only a small amount of the drug 10% - 20% is systemically available. The daily average dose of Marinol ranges between 10 mg - 40 mg and may result in heavy intoxication.

### Vaporisers

Usually vaporisers expose the medium to be vaporised to a controllable heat source and airflow from a built in fan to assist vaporisation. The user, where possible, is encouraged to adjust the temperature to a point just below or at the point of combustion of the given medium such that only vapour is inhaled from the device, thus reducing exposure to the harmful toxins found in smoke at higher temperatures.

Typically a vaporiser uses a heat source and fan assisted airflow to efficiently evaporate compounds in ethanolic solution to ultra-fine vapour particles with 600-800 nanometres in diameter, which appear as a fine white wispy smoke-like vapour. For use the vapour is captured in an inflated reservoir, which stands up to 1 meter above the base unit when fully inflated.

Researchers have found over 4500 toxins within smoke derived from plant material (Dales Gieringer, Joseph St. Laurent, Scott Goodrich Cannabis Vaporizer Combines Effective Suppression of Pyrolytic Compounds. Journal of Cannabis Therapeutics Vol 4(1) 2004). Although there are far fewer toxins within vapour at lower temperatures, it is not free of harmful toxins as it is often assumed to be.

### Sublingual Spray

Manufacture and purification of cannabinoid blends and the delivery thereof via sublingual route are outlined in GB 2377633A and WO 02/064109 A2. The sublingual drug delivery technology results in a faster time to onset and a reduced first pass effect when compared to Marinol ®.

### Inhalers

Preferred inhalers are pressured metered dose inhaler (pMDI), which comprise a container for storing a pressured aerosol composition and an actuator with a valve metering chamber, an orifice for dispensing an accurate dose of the composition and to atomize and create a plume of droplets of the dispensed composition. The composition comprises an active ingredient and a propellant, which provides the energy required to generate atomisation. Inhalers can be adapted for suspension, emulsion or solution preparations.

The propellant typically is an alkane, for example a hydrofluoroalkane (HFA). HFA solution based pMDIs are well known to deliver compounds systemically (Leach CL, Davidson PJ, Hasselquist BE, Boudreau RJ. Deposition Comparison of CFC -Fluticasone, CFC-Beclomethasone, and HFA-Beclamethasone MDIs in Healthy Subjects. Paper published and ERS Stockholm 2002). A major problem in the administration of cannabinoid preparations for inhalation via a pMDI is the reflex coughing induced by cannabinoid inhalation, particularly THC. This cough reaction is subjectively unpleasant and reduces the amount of systemically delivered dose of cannabinoids.

Williams et al (Williams, S J., Hartley, J.P.R., Graham, J.D.P. (1976) Bronchodilatory effect of delta-9-THC administered by aerosol to asthmatic patients. Thorax 31:720-723) described a pMDI containing THC. Although their investigation found some evidence that THC exhibited a bronchodilatory and anti-inflammatory effect upon the lung, it disclosed that inhaled THC caused the patients to cough. Coughing problems also are reported in WO 01/66089 regarding the administration of THC and a propellant to the lungs of a patient. To reduce the above discussed negative side effects in WO 01/66089 a sublingual delivery in an aerosol is proposed.

Alternatively WO 04/000290 discloses a pharmaceutical composition for an aerosol, which comprises a cannabinoid, a propellant and a medium triglyceride or propylene glycol diester as a cough suppressant.

In WO 2005079771 discloses a composition / method for relief of pain comprising the local administration of a subject in need of such treatment of a pharmaceutical composition comprising at least an analgesic like a local anaesthetic (lidocaine, tetracaine) and a cannabinoid. But the term cannabinoid corresponds to endocannabinoids. Furthermore the only disclosure of a cannabinoid from cannabis origin is described as having unwanted side effects.

In all the examples the analogue compound L-29 of D1 is administered by an injection. The inhalation is only one of the possible ways of administering and not of a cannabinoid but of L-29.

Further, in a recent study (Naef M. et al. J. Pharm. Sci. 2004; 93:1176-84) it was shown an increased bioavailability (29%) after inhalation of a THC aerosol by using a pressure-driven inhalator (Pari Master). The tolerability of the aqueous, nebulised aerosol was good but significant cough and irritation of the upper airways was seen influencing the efficiency of the inhalation process and the resulting bioavailability.

Thus, there is a need for a system for a pulmonary administration of cannabinoids, which allows a suppression of the cough reflex usually induced by cannabinoid inhalation.

### Summary of the invention

It is the primary object of the invention to provide means and methods for a pulmonary administration of cannabinoids,

The applicant has found that surprisingly the addition of a local anaesthetic to a composition for pulmonary administration comprising at least one cannabinoid, which alleviates the coughing reflex. Accordingly, the present invention provides a pharmaceutical composition suitable for pulmonary administration comprising at least one cannabinoid, at lease one local anaesthetic and a pharmaceutical acceptable carrier, diluent or excipient.

Furthermore, the applicant found that such addition of a local anaesthetic to a composition suitable for pulmonary administration allows and improves the delivery and bioavailability of an accurate dose and also of repeated doses of cannabinoids. Further such addition of a local anaesthetic to such composition suitable for pulmonary administration even allows the administration of high, however, still pharmaceutically tolerable drug concentrations.

In one embodiment of the invention the composition comprises additionally a propellant and ethanol.

Accordingly, the above stated object is achieved by providing a pharmaceutical composition suitable for pulmonary administration or as an aerosol, which comprises at least one cannabinoid, at least one local anaesthetic, which assists in suppressing a coughing reflex, and a pharmaceutical acceptable carrier, diluent or excipient.

The term "cannabinoid", for the purpose of this invention, includes all major and minor cannnabinoids found in natural cannabis and hemp material that can be isolated and reproduced by synthetic means. This includes delta-9-Tetrahydrocannabinol (THC), delta-8-Tetrahydrocannabinol, Cannabidiol (CBD), Cannabinol (CBN), tetrahydrocannabinovarin (THCV), cannabidivarin (CBDV), cannabigerol (CBG) and cannabichromene (CBC). In addition the term cannabinoid includes synthetic cannabinoids and further combinations and acids of said natural and synthetic cannabinoids.

According to the invention the local anaesthetic is preferably a pharmaceutical acceptable aminoamide or aminoester, preferably selected from the group consisting of Lidocaine, Articaine, Mepivicaine, Etidocaine, Prilocaine, Bupivacaine, Procaine, Tetracaine, Benzocaine and Chloroprocaine and includes the free base of the local anaesthetic and pharmaceutival acceptable salts thereof, and a pharmaceutically acceptable excipient. Typical examples of these salts are the hydrochloride, citrate, tartrate, bensoate and succinate.

According to one embodiment the composition comprises the local anaesthetic in the broad range of 0.1 - 20% w/v, preferably the selected range is between of 0.1 - 1.0% w/v, 0.5 - 1.0% w/v, 0.5 - 2.0% w/v, 0.5 - 5.0% w/v, of 1.0 - 10.0% w/v, 5.0 - 8.0% w/v, 5.0 - 10.0% w/v, 5.0 - 15.0% w/v, 8.0 - 12.0% w/v, 8.0 - 20.0% w/v, 10.0 - 15.0% w/v, 10.0 - 20.0% w/v, 12.0 - 18.0% w/v, 12.0 - 20.0% or w/v, 15.0 - 20.0% w/v.

It is to be acknowledged that it is the achievement of the applicant that the addition of the local anaesthetic in a composition suitable for pulmonary administration comprising cannabinoids was found to be efficient to alleviate and prevent cannabinoid irritation and coughing reflexes and thereby renders tolerable single and also repeated doses of cannabinoids and even high drug concentrations of cannabinoids.

The cough reducing effect of the local anaesthetic in this composition is very surprising, since the general onset time of local anaesthetics is between 2 and 10 minutes and therefore slow in comparison with the onset time of coughing after inhalation of cannabinoids, which is in the range of seconds. Nevertheless, the composition of the invention proofed to be highly effective in alleviating and preventing a cannabionoid induced coughing. Therefore, the composition of the invention is particularly useful for pulmonary administration and thus systemic distribution and bioavail cannabinoid. Furthermore, the composition according to the invention may provide rapid relief for acute pain and subsequent maintenance of breakthrough pain.

It should be noted that the invention also provides the use of a local anaestetic in the manufacturing of a medicament for pulmonary administration, wherein the local anaestetic prevents and/or alleviates a reflex coughing due to irritations -also comprising other than cannabinoid induced irritations - of the administered aerosol.

According to further embodiments the weight ration of the local anaesthetic to the cannabinoid is between 1:2 and 4:1, alternatively it is between 1:1 and 3:1, further alternarively it is between 2:1 and 2,5: 1. According to a selected embodiment the ratio to the local anaesthetic to THC is between 2:1 or 3:1, however the THC amount is preferably above 200 mcg THC per actuation.

According to another embodiment the composition can be prepared a suspension or a solution preparation of the cannabinoid. The skilled person, a pharmacologist, knows about several methods to on one hand solve the active ingredients of interest as well as formulate them in a stable solution or suspension.

In one embodiment of the invention the composition comprises additionally ethanol. Cannabinoid compounds or combinations thereof are lipophilic and are generally soluble up to 20 - 30 % in absolute ethanol. Therefore, it is suggested that the composition comprises as co-solvent a certain amount of ethanol. Accordingly the composition of the invention comprises in one further embodiment ethanol in the range of 1% to 30% v/v, preferably the selected range is between 1% to 5% v/v, 2% to 8% v/v, 5% to 10% v/v, 8% to 15% v/v, 10% to 20% v/v, 15% to 25% v/v, 18% to 27% v/v or 18% to 30% v/v.

An ethanol concentration of above 50% should be avoided, since high ethanol content is regarded as undesirable as it increases particle size and velocity in the aerosol and therefore reducing the lung deposition of the aerosol.

It is noted that also other suitable co-solvents, which are known to the skilled person, are included in the scope of the invention.

In still a further embodiment the composition of the invention comprises additionally a propellant to improve the aerosol characteristics of the composition and thereby providing a composition which is even more suitable for pulmonary administration.

In further embodiments of the present invention, the pharmaceutical composition comprises alternatively or additionally a polyhydric alcohol additionally useful for cough repression. Preferably, but not limited, the polyhydric alcohols are water-soluble.

In one embodiment the pharmaceutical composition comprise preferably from 0.01 to 0.1 % by weight of polyhydric alcohol. Preferred is a pharmaceutical composition which comprises glycerol (1,2,3-trihydroxy-propane or propan-1,2,3-triol) by weight from 0.01 to 0.1%. The polyhydric alcohols are usually used in the form of a hydrous product. In this case, the weight ratio of the water-soluble polyhydric alcohol and water is preferably in the range of 95:5 to 50:50, and especially 90:10 to 55:45.

Examples of the polyhydric alcohol to be used in the present invention are ethylene glycol, propylene glycol, 1,3-butylene glycol, 1,4-butylene glycol, dipropylene glycol, polypropylene glycol, glycerol, diglycerol, polyglycerol, polyethylene glycol, erythritol, xylytol, sorbitol, maltitol, lactitol, mannitol, trehalose, sugar alcohol derived from digestion product of starch and the like. Among these polyhydric alcohols, especially preferred ones are glycerol, diglycerol, polyglycerol and sorbitol.

According to a further embodiment a composition with additional glycerol content of 0.01 - 0.1% w/w also reduces reflex coughing. It is however possible to distinguish between a glycerol-containing and a glycerol-free formulation in regard of cough repression, and it was furthermore shown that the composition with the local anaesthetic is more effective than the composition containing glycerol.

According to the invention for administration of the pharmaceutical composition preferably a metered dose dispenser, especially a metered dose inhaler is used, although other inhaler devices are also included in the scope of the invention. Therefore, according to a preferred aspect of the invention a metered dose dispenser or inhaler is provided containing a pharmaceutical composition according to the invention. The preferred metered unit dose is containing 0.05 to 0.5 mg, preferably from 0.1 to 0.2 mg of the cannabinoid.

The composition of the invention is particularly useful to be administered by an inhaler.

There are several parameters which influence lung and throat deposition of the aerosol which are typically plume velocity, plume shape and particle size, the latter often referred to as mass median aerodynamic diameter (MMAD). Said parameters may include or depend on vapour pressure, the size of valve metering chamber and the actuator orifice geometry.

The atomization characteristics of an aerosol plume can be optimized to target a desired region of the lung for a given formulation.

The aforementioned parameters influence the key parameter, the fine particle dose (FPD). This is the amount of emitted dose that actually reaches the lung and is expressed as the mass of the ex valve dose less than or equal to 4.7 microns. This can also be expressed as a fraction of the ex valve dose less than or equal to 4.7 microns, fine particle fraction (FPF). To achieve high systemic delivery, the fine particle fraction (FPF) should be as high as possible within predefined limits.

In general terms, as the orifice diameter decreases, atomisation efficiency improves, raising FPD. As one would expect, reducing orifice diameter increases the duration of the plume event whilst reducing the plume velocity.

Orifice geometry is optimised for a given composition to achieve a desired plume characteristics and lung deposition whilst avoiding clogging during use. The formulation and the actuator characteristics both play a role in the plume dynamics, which influence lung deposition.

The energy required to generate atomisation is provided by the propellant and is therefore an additional parameter to influence plume characteristics.

Preferred HFA propellants are 134a and 227, which have different vapour pressures thus enabling to blend propellants to achieve optimum vapour pressure and plume characteristics.

Further, it is found that the inclusion of a low glycerol content preferably 0.01 - 0.1% w/w improves atomization and clogging resistance particularly at concentrations. The use of glycerol is especially favorably in conjunction with the use of small orifice diameter actuators < 0.22mm and particularly 014 - 0.18mm.

A typical pMDI plume is reliant upon propellant driven atomisation generating a MMAD 1 - 5 microns with a plume velocity up to 28m/sec. Maximum velocity occurs at the beginning of the plume event, close to the actuator with a typical plume duration of 200ms.

Preferably the plume should contain as much particulate as possible within a predetermined particle size range (800 - 1100 nm) such that the amount of emitted dose available for systemic absorption is maximised.

This range is at the lower limit of pMDI plume generation and in reality only a very small percentage of the emitted dose from conventional pMDIs is close to this range. Most pMDIs have a MMAD in the range 1.1 - 3.5 microns.

Particulate less than 0.8 microns MMAD are exhaled by the patient and if too large, greater than 5 microns will impact upon the back the throat and will fail to reach the target area of the lung in any significant quantity.

The applicant has observed that optimal deep lung deposition is achieved when the plume velocity of a pMDI approaches the velocity of a patient's natural inspiratory flow, typically generated between 10-15 m/sec. Plume velocities 30% lower than conventional pMDIs are prefered whereby the measurement are made with imaging velocimetry (TSI Instruments) with small orifice diameters < 0.22mm and particularly 014 - 0.18mm down to values as low as 1m/sec. The effect of decreasing plume velocity reduces turbulence and therefore throat deposition. In addition the dependency of lung deposition upon patient device co-ordination is reduced for reduced plume velocities.

Preferably the output characteristics of a pMDI for systemic absorption of cannabinoids are
- a slow soft plume 1 - 3m/sec
- at a point 50- 100mm from the actuator mouth
- containing particulate between 0.8 -1.0 microns MMAD
- over a plume duration of 1.5 - 3 seconds.

### Pharmaceutical formulations

Cannabinoid containing formulations according to the invention were prepared for *in vitro* testing to establish the influence of ethanol, cannabinoid and Tetracaine content upon Fine Particle Fraction.

Canisters were filled to 5-10 ml volume with HFA 227 propellant and crimped with a 50 mcI valve. In examples 1 - 4 formulae containing a range of cannabinoid options in a 20% w/v ethanolic solution, a glycerol 2.5% (w/v) solution and Tretacain free base (20% w/v) solution to the following approximate proportions. A test actuator was used from Bespak with an orifice diameter of 0.22mm.

In the following certain aspects of the invention will be described in more details for selected examples. It will be understood that the examples have been provided to illustrate the invention. The invention is not limited to compositions using the particular local anaesthetics, cannabinoids or propellants described in these examples or particularly described herein. Following the teachings herein about how the cough reflex may be suppressed in aerosol formulations containing a cannabinoid and a propellant, those skilled in the art should readily be able to identify other local anaesthetics for cough suppression, other propellants and other co-solvents.

### Example 1

### Contents per canister :

Cannabinoids : 5mg THC and 5mg CBD
Glycerol : 0.125mcg
Overall ethanol 2% v/v
HFA 227 98% v/v

Dose per actuation 50mcg THC + 50mcg CBD + 25mcg Tretacain

### Example 2

### Contents per canister:

Cannabinoid : 20mg THC and 20mg CBD.
Glycerol 1.25mg
Overall Ethanol content 5% v/v
HFA 227 95% v/v

Dose per actuation 200mcg THC + 200mcg CBD

### Example 3

### Contents per canister:

Cannabinoids : 20mg THC and 20mg CBD
Tretracin 40mg
Glycerol 1.25mg
Overall Ethanol content 9% v/v
HFA 227 propellant 91% v/v

Dose per actuation 200mcg THC + 200mcg CDB + 400mcg Tetracain.

### Example 4 THCV and CBD with Tretracain

### Contents per canister

Cannabinoids : 10mg THVC and 10mg CBD
Tretacaine 20mg
Glycerol 1.25mg
Overall ethanol content 5% v/v
HFA Propellant 227 95 v/v

Dose per actuation 100mcg THCV + 100mcg CBD + 200mcg Tetracaine

**Table 1**

| | MMAD (microns) | Fine Particle Fraction (%) at <4.7um |
|---|---|---|
| Example1 | 1.0THC | 79 THC |
| | 1.0 CBD | 79 CBD |
| Example2 | 1.5 THC | 73 THC |
| | 1.5 CBD | 72 CBD |
| Example3 | 1.9 THC | 67 THC |
| | 1.9 CBD | 67 CBD |
| Example4 | 1.5THCV | 73 THCV |
| | 1.5 CBD | 73 CBD |

Shot weights remained consistent and there is as no evidence of actuator clogging in any of the preparations.

Both THC and CBD have similar volumes of distribution and deposition profiles suggesting that that compounds may be systemically absorbed in similar quantities when administered equally. The inclusion of Tretracain does not effect the even deposition profile of THC and CBD other than the overall detrimental effect of a raised ethanol content.

As one would expect there is an increase FPF as ethanol content is reduced and a reduction in MMAD from 1.9 for 9 % ethanol to 1.0um for 2% ethanol content. Fine particle fraction was recorded as 67% for 9% ethanol and 79% for 2% ethanol with a cut off at 4.7microns.

Results were obtained using an impactor which is described by Mitchell et.al. (Jolyon P.Mitchell, Mark W. Nagel, Kimberly J.Wiersemma and Cathy C. Dole Aerodynamic Particle Size Analysis of Aerosols from Pressurized Metered Dose Inhalers:Comparison of Anderson 8-Stage Cascade Impactor, Next Generation Pharmaceutical Impactor and Model 3321 Aerodynamic Particle Sizer Aerosol Spectrometer.AAPSPharmSciTech2003:4(4)Article54(http://www.aapspharmscitech.org) ref 15. Raw data was processed using Copley Inhaler Testing Analysis Software (CITAS).

Further cannabinoid formulations with a range of Lidocaine HCL, Lidocaine free base and Tetracaine free base and with ethanol, glycerol, HFA 227 were tested for solubility and effectiveness. The results are displayed in tables 2 - 5.

**Table 2**

| **Range of contents with Lidocaine HCL** | | | | | |
|---|---|---|---|---|---|
| **Ethanol** | **Glycerol** | **HFA 227** | **THC** | **CBD** | **Lid HCL** |
| (mg) | (mg) | (mg) | (mg) | (mg) | (mg) |
| 50 - 400 | 0.1 - 2 | 1000 - 12000 | 0.1 - 50 | 0.1 - 50 | 1 - 60 |

| **Preferred example of formulation with Lidocaine HCL** | | | | | |
|---|---|---|---|---|---|
| (mg) | (mg) | (mg) | (mg) | (mg) | (mg) |
| 400 | 1 | 7500 | 20 | 20 | 40 |

| | | | | | |
|---|---|---|---|---|---|
| Upper effective ratio for Lid HCL 60 mg per 20mg THC ( 3;1 )* Optimal ratio 40mg per 20mg 2 : 1 Minimum observed effective ratio 20mg per 20mg 1 :1 ** * Excessive anaesthetic effect above this ratio ** Coughing not suppressed below this ratio Limit of solubility of Lid HCL in Ethanol 200mg/ml Limit of solubility of Lid HCL in HFA 227 4mg/ml | | | | | |

**Table 3**

| **Range of contents with Lidocaine Base** | | | | | |
|---|---|---|---|---|---|
| **Ethanol** | **Glycerol** | **HFA 227** | **THC** | **CBD** | **Lid Base** |
| (mg) | (mg) | (mg) | (mg) | (mg) | (mg) |
| 50 - 350 | 0.1-2 | 1000 - 12000 | 0.1 - 50 | 0.1 - 50 | 1 - 60 |

| **Preferred example of formulation with Lidocaine Base** | | | | | |
|---|---|---|---|---|---|
| (mg) | (mg) | (mg) | (mg) | (mg) | (mg) |
| 350 | 1 | 7500 | 20 | 20 | 20 |

| | | | | | |
|---|---|---|---|---|---|
| Upper effective ratio for Lid base 60 mg per 20mg THC (3;1)* Optimal effective ratio 40mg per 20mg 2:1 Minimum ratio 20mg per 20mg 1:1** Limit of solubility of Lid Base in Ethanol : 700mg/ml Limit of solubility of Lid Base in HFA 227 : 80mg/ml | | | | | |

**Table 4**

| **Range of contents per cansiter with Tetracaine Base** | | | | |
|---|---|---|---|---|
| **Ethanol** | **HFA 227** | **THC** | **CBD** | **Tet** |
| (mg) | (mg) | (mg) | (mg) | (mg) |
| 50 - 350 | 1000 - 12000 | 0.1 - 50 | 0.1 - 50 | 1 - 60 |

| **Preffered example of formulation with Tetracaine Base** | | | | |
|---|---|---|---|---|
| (mg) | (mg) | (mg) | (mg) | (mg) |
| 350 | 7500 | 20 | 20 | 20 |

| | | | | |
|---|---|---|---|---|
| Upper effective ratio for Tet 40 mg per 20mg THC ( 2:1 )* Optimal effective ratio 20mg per 20mg 1:1 Minimum effective ratio 5mg per 20mg 0.25 :1 ** Note : Glycerol found not to be required with Tretracaine Base Limit of solubility of Tetracain in Ethanol : 450mg/ml Limit of solubility of Tetracain in HFA 227 : 40mg/ml | | | | |

**Table 5**

| **Range of contents per cansiter with Tetracaine Base CBD only** | | | |
|---|---|---|---|
| **Ethanol** | **HFA 227** | **CBD** | **Tet** |
| (mg) | (mg) | (mg) | (mg) |
| 10 - 350 | 1000 - 12000 | 0.1 - 50 | 1 - 45 |

| **Preferred example of formulation with Tetracaine Base** | | | |
|---|---|---|---|
| (mg) | (mg) | (mg) | (mg) |
| 80 | 7500 | 20 | 15 |

| | | | |
|---|---|---|---|
| Upper effective ratio for Tet 20 mg per 20mg CBD ( 1:1 )* Optimal effective ratio 15mg Tet per 20mg CBD (0.75 : 1) Minimum effective ratio 5mg per 20mg 0.25:1 ** Note : Glycerol found not to be required with Tretracaine Base Limit of solubility of Tetracain in Ethanol : 450mg/ml Limit of solubility of Tetracain in HFA 227 : 40mg/ml | | | |

### Clinical tests

In an open study on 12 healthy volunteers a combination of THC and cannabidiol (CBD) was compared after inhalation (i.h.) and intravenously (i.v.) (see Rudolf Brenneisen, Pascale Meyer, Manuela Langos, Christian Steup and Andrew Davies, IACM Congress 2007, 05.10.2007 Cologne).

The liquid aerosol was produced by an in-vitro validated pMDI device, releasing about 45% of the cannabinoid dose, enabling a dosage of about 90 mcg THC and 90 mcg CBD per actuation. The actuator was used from Bespak with an orifice diameter of 0.22mm.

Three subjects (pilot study) received 360 and 9 subjects 720 mcg THC-CBD, corresponding to 4 and 8 actuations, respectively. The addition of a Lidocain HCL to the pulmonal preparation was expected to prevent mucosa irritation and coughing. The pharmacokinetic evaluation was based on plasma profiles aquired by GC/MS. Adverse effects were monitored by visual analog scales (VAS) and measuring vital functions.

After 360 mcg i.h. and i.v. doses, THC and CBD were not measurable in plasma longer than 20 min after administration, therefore only plasma levels resulting after 720 mcg were further evaluated. After i.h. and i.v. administration, THC plasma peaks were observed 5 min post-drug, with THC peak concentrations from 3 to 22 and 13 to 40 ng/ml, respectively. CBD peaks, with concentrations from 2 to 17 and 14 to 26 ng/ml, were also measured after 5 min. The elimination half-lives were 7 (THC) and 11 min (CBD) after i.h., 22 and 24 min after i.v. administration. The mean i.h. bioavailability (calculated vs. i.v.) was 55 ± 37 and 59 ± 47 % for THC and CBD, respectively. This represents a 2-fold increase compared to the previous study of Naef et.al. After i.h., the THC metabolism was less pronounced than after oral ( p.o.) administration. Conjugated 11-carboxy-THC was the main metabolite. The aerosol was generally well tolerated with little or no coughing, and only slight psychotropic side-effects were observed in some subjects. These were more distinct after i.v., especially irritations and hallucinations. Besides moderate tachycardia, the vital functions stayed unchanged.

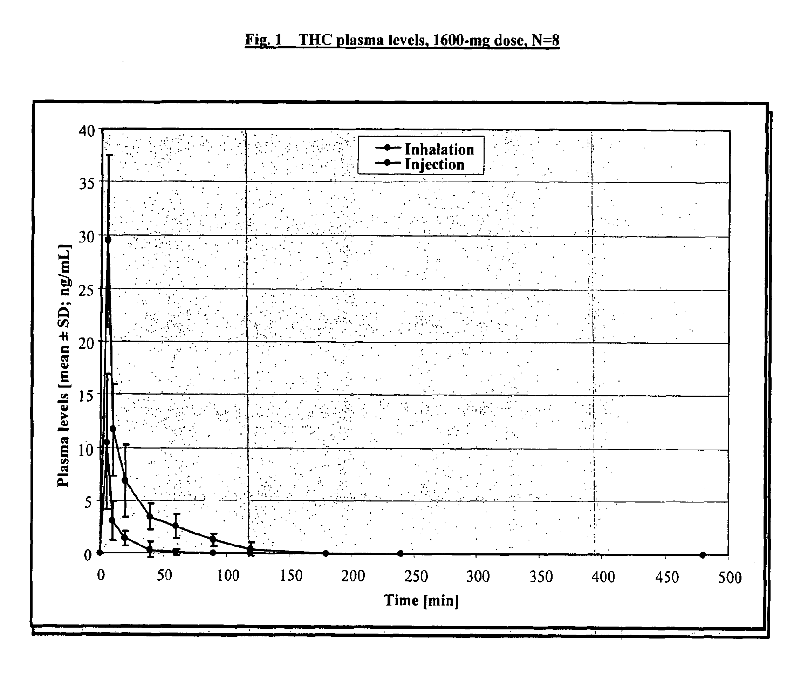

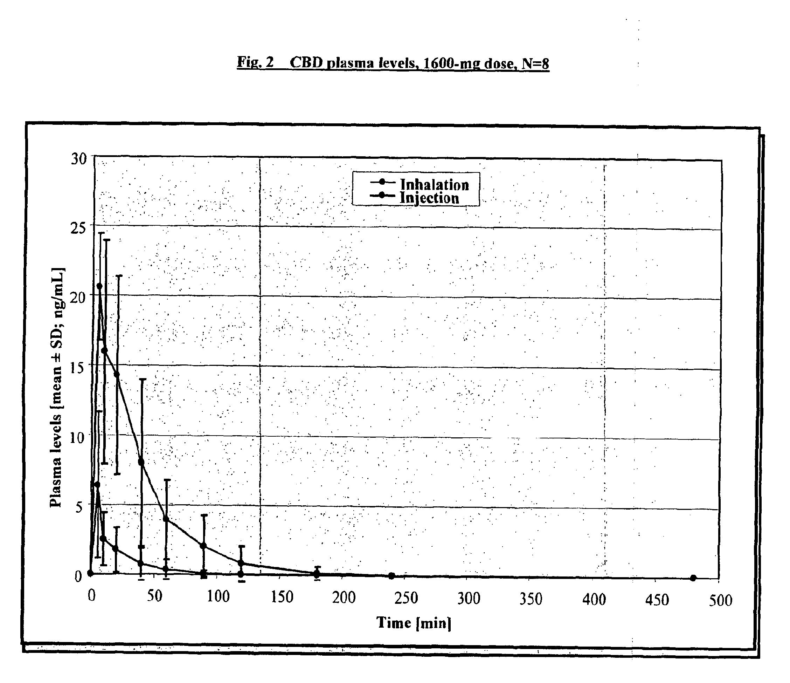

In figures 1 and 2 the time dependant THC and CBD plasma levels for a 1600mg dose are displayed for i,h, and i.v. administration for comparison.

Conclusion is that a THC-CBD i.h. aerosol shows favorable pharmacokinetic properties, which are similar to those of an i.v. preparation. Adding a local anaesthetic is recommended to prevent coughing. The negligible psychoactivity may result from the antipsychotic CBD, the low THC dosage and/or the decreased formation of the psychoactive metabolite 11-hydroxy-THC. Therefore, the inhalation alternative to the p.o. administration route and is an option for reliable and safe application of cannabinoids in a clinical context.

### Results

An inhaler containing a composition comprising cannabinoid compounds, THC and/or CBD, a HFA propellant, low glycerol content is effective for symptom relief of a range of ailments. The composition is administered via an actuator containing a small orifice diameter suitable for use with high ethanol content. Actuation of the inhaler generates high fine particle fraction (FPF) suitable for systemic delivery. Furthermore coughing associated with cannabinoid inhalation is overcome with the inclusion of lidocaine or similar local anaesthetics rendering high and repeat cannabinoid doses tolerable. The inclusion of lidocaine and similar local anaesthetics is in a range of 0.1 - 20% w/v to relieve reflex coughing caused by cannabinoid inhalation.

The pMDI provides means of delivering cannabinoids in therapeutically beneficial serum concentrations 0 -135ng/ml within 10 minutes.

Preferred is the use of THC CBD in a pMDI or other inhalation devices within the range 0.01 - 20% w/v THC and 0.01 - 20 w/v % CBD used separately or in combination thereof.

### References

1. C.H.Ashton et al (2005);"Cannabinoids in bipolar affective disorder: a review and discussion of their therapeutic potential" Psychopharm 2005
2. Tashkin, D.P. Effects of Marijuana on the Lung and its Immune Defences. Indiana Prevention Resource Centre March 1997.
3. Dales Gieringer, Joseph St. Laurent,Scott Goodrich Cannabis Vaporizer Combines Effective Suppression of Pyrolytic Compounds. Journal of Cannabis Therapeutics Vol 4(1) 2004
4. Output characteristics of Dronabinol vaporised in the Volcano. STI Pharmaceuticals Ltd Internal Report 0020/2004
5. Myrtha Naef ,Stefan Russman ,Steen Petersen-Felix, Rudolf Brenniesen: Development and Pharmacokinetic Characterisation of Pulmonal and Intravenous Delta -9 Tetrahydrocannabinol (THC) in Humans. Journal of Pharmaceutical Science Vol 93 No 5 May 2004
6. Mahmoud A. ElSohly A Suppository Formulation for Delivery of Delta 9 Using a Prodrug. Paper presented to ICAM 2003 conference Cologne Medical School.
7. Williams, S J., Hartley, J.P.R., Graham, J.D.P. (1976) Bronchodilatory effect of delta-9-THC administered by aerosol to asthmatic patients. Thorax 31:720-723
8. Leach CL, Davidson PJ, Hasselquist BE, Boudreau RJ. Deposition Comparison of CFC -Fluticasone, CFC-Beclomethasone, and HFA-Beclamethasone MDIs in Healthy Subjects. Paper published and ERS Stockholm 2002
9. Busse W, Colice G, Hannon S. CFC-BDP requires 2.6 times the dose to achieve equivalent improvement FEV1 as HFA-BDP. Am J Respiratory Crit Care Med 1998; 49:A405
10. Stephen W. Stein, James S. Stefely. Reinventing Metered Dose Inhalers : From poorly Efficient CFC MDIs to Highly Efficient HFA MDIs. Drug Delivery Technology Jan/Feb2003 Vol 3 No 1
11. D.A. Lewis, S Johnson et al Effects of Actuator Orifice Diameter on Beclomethasone Dipropionate Delivery From a pMDI HFA Solution formulation. Respiratory Drug Delivery VI, 1998
12. D. Ganerton, D Lewis,R Davies, B Meakin G Brambilla, T. Church. Modulite : a means of designing the aerosols generated by pressurised metered dose inhalers. Respiratory Medicine August 2002 Vol 96
13. R.J. Davies, D.A. Lewis, D. Ganderton, B. J. Meakin, G. Brambilla, S.D. Murphy and T.R. Nicholls. Velocity profiling of a new HFA Budesonide pMDI : Respiratory Drug Delivery VIII 2002.
14. G Brambilla R.J Davies, A Ferraris, D Ganderton, F Keay, D. A. Lewis, B.J Meakin, S,D Murphy,T.r. Nicholls. Plume Profiling of Beclomethasone Dipropionate pMDIs
15. Harald Groeben et al Combined Lidocaine and Salbutamol Inhalation for Airway Anaesthesia Markedly Protects Against Bronchoconstriction Chest 2000; 118:509-515
16. Jolyon P.Mitchell, Mark W. Nagel, Kimberly J.Wiersemma and Cathy C. Dole Aerodynamic Particle Size Analysis of Aerosols from Pressurized Metered Dose Inhalers:Comparison of Anderson 8-Stage Cascade Impactor, Next Generation Pharmaceutical Impactor and Model 3321 Aerodynamic Particle Sizer Aerosol Spectrometer.AAPSPharmSciTech2003:4(4)Article54(http://www.aapspharmscitech. org)
17. Cannabinoids in bipolar affective disorder: a review and discussion of their therapeutic potential. C.H.Ashton et al Psychopharm 2005.

## Claims

1. A pharmaceutical composition suitable for pulmonary administration of a cannabinoid, comprising at least one cannabinoid, at least one local anaesthetic and a pharmaceutical acceptable carrier, diluent or excipient, wherein the cannabinoid is selected from the group consisting of delta-9-Tetrahydrocannabinol (THC), delta-8-Tetrahydrocannabinol, Cannabidiol (CBD), Cannabinol (CBN), tetrahydrocannabinovarin (THCV), cannabidivarin (CBDV), cannabigerol (CBG) or cannabichromene (CBC) and their pharmaceutically acceptable salts or acids.

2. A composition as claimed in claim 1, wherein the local anaesthetic is an aminoamide or aminoester, preferably selected from the group consisting of Mepivicaine, Etidocaine, Articaine, Lidocaine, Prilocaine, Bupivacaine, Procaine, Tetracaine, Benzocaine and Chloroprocaine and pharmaceutical acceptable salts thereof.

3. A composition as claimed in any of the claims 1 or 2, which comprises the local anaesthetic in a range of 0.1 - 20% w/v.

4. A composition as claimed in any one of the claims 1 to 3, wherein the weight ratio of the local anaesthetic to cannabinolds is in the range of from 1:2 to 4:1.

5. A composition as claimed in claim 4, in which the weight ratio of local anaesthetic to cannabinoid is in the range of from 1:1 to 3:1.

6. A composition as claimed in claim 5, wherein the weight ratio of local anaesthetic to cannabinoid is in the range of from 2:1 to 2,5:1.

7. A composition as claimed in any one of the claims 1 to 6, comprising additionally ethanol in the range from 1 % to 20% v/v.

8. A composition as claimed in any one of the claims 1 to 7, comprising additionally a propellant, wherein the propellant is 1,1,1,2-tetrafluoroethane or 1,1,1,2,3,3,3-heptafluoropropane.

9. A composition as claimed in any one of the claims 1 to 8, additionally comprising a polyhydric alcohol, preferably glycerol or propylene glycol.

10. A composition as claimed in claim 9, which comprises from 0.01 to 0.1% by weight of glycerol.

11. An inhaler containing the pharmaceutical composition as claimed in any one of claims 1 to 10.

12. An inhaler as claimed in claim 11, which is adapted to provide a unit dose containing from 0.05 mg to 5 mg of cannabinoid.

13. The use of a pharmaceutical composition comprising at least one cannabinoid found in natural cannabis and hemp material that can be isolated or reproduced by synthetic means, at least one local anaesthetic and a pharmaceutical acceptable carrier, diluent or excipient in the manufacture of a medicament for pulmonary administration to prevent and/or alleviate a reflex coughing due to irritations of the administered aerosol..

14. The use as claimed in claim 13, wherein the cannabinoid is selected from the group consisting of delta-9-Tetrahydrocannabinol (THC), delta-8- Tetrahydrocannabinol, Cannabidiol (CBD), Cannabinol (CBN), tetrahydrocannabinovarin (THCV), cannabidivarin (CBDV), cannabigerol (CBG) or cannabichromene (CBC) and their pharmaceutically acceptable salts or acids.

## Patentansprüche

1. Pharmazeutische Zusammensetzung, die sich für die pulmonale Verabreichung eines Cannabinoids eignet, umfassend mindestens ein Cannabinoid, mindestens ein Lokalanästhetikum und einen pharmazeutisch unbedenklichen Träger, ein pharmazeutisch unbedenkliches Verdünnungsmittel oder einen pharmazeutisch unbedenklichen Grundstoff, wobei das Cannabinoid aus der Gruppe bestehend aus delta-9-Tetrahydrocannabinol (THC), delta-8-Tetrahydrocannabinol, Cannabidiol (CBD), Cannabinol (CBN), Tetrahydrocannabinovarin (THCV), Cannabidivarin (CBDV), Cannabigerol (CBG) oder Cannabichromen (CBC) und ihren pharmazeutisch unbedenklichen Salzen oder Säuren ausgewählt ist.

2. Zusammensetzung nach Anspruch 1, wobei es sich bei dem Lokalanästhetikum um ein Aminoamid oder einen Aminoester, vorzugsweise ausgewählt aus der Gruppe bestehend aus Mepivicain, Etidocain, Articain, Lidocain, Prilocain, Bupivacain, Procain, Tetracain, Benzocain und Chlorprocain und ihren pharmazeutisch unbedenklichen Salzen, handelt.

3. Zusammensetzung nach einem der Ansprüche 1 oder 2, die das Lokalanästhetikum in einem Bereich von 0,1 - 20% w/v umfasst.

4. Zusammensetzung nach einem der Ansprüche 1 bis 3, wobei das Gewichtsverhältnis von Lokalanästhetikum zu Cannabinoiden im Bereich von 1:2 bis 4:1 liegt.

5. Zusammensetzung nach Anspruch 4, in der das Gewichtsverhältnis von Lokalanästhetikum zu Cannabinoiden im Bereich von 1:1 bis 3:1 liegt.

6. Zusammensetzung nach Anspruch 5, in der das Gewichtsverhältnis von Lokalanästhetikum zu Cannabinoiden im Bereich von 2:1 bis 2,5:1 liegt.

7. Zusammensetzung nach einem der Ansprüche 1 bis 6, die zusätzlich Ethanol im Bereich von 1% - 20% v/v umfasst.

8. Zusammensetzung nach einem der Ansprüche 1 bis 7, die zusätzlich ein Treibmittel umfasst, wobei es sich bei dem Treibmittel um 1,1,1,2-Tetrafluorethan oder 1,1,1,2,3,3,3-Heptafluorpropan handelt.

9. Zusammensetzung nach einem der Ansprüche 1 bis 8, die zusätzlich einen mehrwertigen Alkohol, vorzugsweise Glycerin oder Propylenglykol, umfasst.

10. Zusammensetzung nach Anspruch 9, die 0,01 bis 0,1 Gew.-% Glycerin umfasst.

11. Inhalator, der die pharmazeutische Zusammensetzung nach einem der Ansprüche 1-10 umfasst.

12. Inhalator nach Anspruch 11, der so eingestellt ist, dass er eine Einzeldosis, die 0,05 mg bis 5 mg Cannabinoid enthält, bereitstellt.

13. Verwendung einer pharmazeutischen Zusammensetzung, die mindestens ein in natürlichem Cannabis- und Hanfmaterial vorkommendes Cannabinoid, das auf synthetische Weise isoliert oder reproduziert werden kann, mindestens ein Lokalanästhetikum und einen pharmazeutisch unbedenklichen Träger, ein pharmazeutisch unbedenkliches Verdünnungsmittel oder einen pharmazeutisch unbedenklichen Grundstoff umfasst, in der Herstellung eines Arzneimittels für die pulmonale Verabreichung zur Vorbeugung und/oder Linderung eines Reflexhustens, der durch Reizungen aufgrund des verabreichten Aerosols verursacht ist.

14. Verwendung nach Anspruch 13, wobei das Cannabinoid aus der Gruppe bestehend aus delta-9-Tetrahydrocannabinol (THC), delta-8-Tetrahydrocannabinol, Cannabidiol (CBD), Cannabinol (CBN), Tetrahydrocannabinovarin (THCV), Cannabidivarin (CBDV), Cannabigerol (CBG) oder Cannabichromen (CBC) und ihren pharmazeutisch unbedenklichen Salzen oder Säuren ausgewählt ist.

## Revendications

1. Composition pharmaceutique adaptée pour administration pulmonaire d'un cannabinoïde, comprenant au moins un cannabinoïde, au moins un anesthésiant local et un véhicule, diluant ou excipient pharmaceutique acceptable, le cannabinoïde étant choisi dans le groupe constitué du delta-9-tétrahydrocannabinol (THC), du delta-8-tétrahydrocannabinol, du cannabidiol (CBD), du cannabinol (CBN), de la tétrahydrocannabinovarine (THCV), de la cannabidivarine (CBDV), du cannabigérol (CBG) ou du cannabichromène (CBC) et leurs sels ou acides pharmaceutiquement acceptables.

2. Composition selon la revendication 1, l'anesthésiant local étant un aminoamide ou un aminoester, de préférence choisi dans le groupe constitué de la mépivicaïne, l'étidocaïne, l'articaïne, la lidocaïne, la prilocaïne, la bupivicaïne, la procaïne, la tétracaïne, la benzocaïne et la chloroprocaïne et des sels ou acides pharmaceutiques acceptables de ceux-ci.

3. Composition selon l'une quelconque des revendications 1 ou 2, qui comprend l'anesthésiant local dans une plage de 0,1 à 20 % m/v.

4. Composition selon l'une quelconque des revendications 1 à 3, dans laquelle le rapport en poids de l'anesthésiant local aux cannabinoïdes est dans la plage de 1:2 à 4:1.

5. Composition selon la revendication 4, dans laquelle le rapport en poids de l'anesthésiant local au cannabinoïde est dans la plage de 1:1 à 3:1.

6. Composition selon la revendication 5, dans laquelle le rapport en poids de l'anesthésiant local au cannabinoïde est dans la plage de 2:1 à 2,5:1.

7. Composition selon l'une quelconque des revendications 1 à 6, comprenant en outre de l'éthanol dans la plage de 1 % à 20 % v/v.

8. Composition selon l'une quelconque des revendications 1 à 7, comprenant en outre un propulseur, le propulseur étant le 1,1,1,2-tétrafluoroéthane ou le 1,1,1,2,3,3,3-heptafluoropropane.

9. Composition selon l'une quelconque des revendications 1 à 8, comprenant en outre un polyol, de préférence le glycérol ou le propylèneglycol.

10. Composition selon la revendication 9, qui comprend de 0,01 à 0,1 % en poids de glycérol.

11. Inhalateur contenant la composition pharmaceutique selon l'une quelconque des revendications 1 à 10.

12. Inhalateur selon la revendication 11, qui est adapté pour fournir une dose unitaire contenant de 0,05 mg à 5 mg de cannabinoïde.

13. Utilisation d'une composition pharmaceutique comprenant au moins un cannabinoïde présent dans le et le matériau naturel de cannabis et de chanvre qui peut être isolé ou reproduit par des moyens synthétiques, au moins un anesthésiant local et un véhicule, diluant ou excipient pharmaceutique acceptable dans la fabrication d'un médicament pour administration pulmonaire pour prévenir et/ou soulager une toux réflexe due à des irritations de l'aérosol administré.

14. Utilisation selon la revendication 13, le cannabinoïde étant choisi dans le groupe constitué du delta-9-tétrahydrocannabinol (THC), du delta-8-tétrahydrocannabinol, du cannabidiol (CBD), du cannabinol (CBN), de la tétrahydrocannabinovarine (THCV), de la cannabidivarine (CBDV), du cannabigérol (CBG) ou du cannabichromène (CBC) et leurs sels ou acides pharmaceutiquement acceptables.
